(19) Europäisches Patentamt / European Patent Office / Office européen des brevets

(11) **EP 2 995 259 A1**

(12) **EUROPEAN PATENT APPLICATION**
published in accordance with Art. 153(4) EPC

(43) Date of publication:
**16.03.2016 Bulletin 2016/11**

(51) Int Cl.:
**A61B 8/14** *(2006.01)*

(21) Application number: **13883993.1**

(22) Date of filing: **10.05.2013**

(86) International application number:
**PCT/KR2013/004126**

(87) International publication number:
**WO 2014/181902 (13.11.2014 Gazette 2014/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.05.2013 KR 20130052724**

(71) Applicant: **Alpinion Medical Systems Co., Ltd.**
**Hwaseong-si, Gyeonggi-do 445-380 (KR)**

(72) Inventor: **LEE, Hyunsook**
**Gyeonggi-do 429-240 (KR)**

(74) Representative: **Thorniley, Peter et al**
**Kilburn & Strode LLP**
**20 Red Lion Street**
**London WC1R 4PJ (GB)**

(54) **ULTRASOUND OPTIMIZATION METHOD AND ULTRASONIC MEDICAL DEVICE THEREFOR**

(57) Some embodiments of the present disclosure provide an ultrasonic optimization method and an ultrasonic medical apparatus therefor. The ultrasonic optimization method and the ultrasonic medical apparatus therefor optimize a transmit parameter of an ultrasound or compensate for a receive parameter thereof according to subject (human body) features identified by analyzing an echo signal of an impulse transmitted to the subject.

*FIG. 8*

**Description**

[Technical Field]

Some embodiments of the present disclosure relate to an ultrasonic optimization method and an ultrasonic medical apparatus therefor. More particularly, some embodiments of the present disclosure relate to an ultrasonic optimization method and an ultrasonic medical apparatus therefor, which identify subject (human body) features by analyzing an echo signal corresponding to an impulse transmitted to a target subject, and optimize a transmit parameter of an ultrasound or compensate for a receive parameter according to the subject (human body) features.

[Background]

The statements in this section merely provide background information related to some embodiments of the present disclosure and do not necessarily constitute prior art.

An ultrasonic apparatus has non-invasive and non-destructive features and thus is widely used in the medical field for obtaining information on the inside of a subject. With the ultrasonic apparatus, a high resolution image of the inside of a subject can be provided in real time without performing a direct surgical incision of the subject for observation. Such an ultrasonic apparatus transmits an ultrasound to the subject and receives an echo signal from the subject to form an ultrasound image of the subject.

Different subject (human body) features inevitably lead to deviations in the ultrasound image which is difficult to be optimized to various human body features. In order to optimize the ultrasound image, an operator needs to directly adjust or manually input image parameters.

[Disclosure]

[Technical Problem]

Therefore, some embodiments of the present disclosure provide an ultrasonic optimization method and an ultrasonic medical apparatus therefor, which is capable of identifying subject (human body) features by analyzing an echo signal corresponding to an impulse transmitted to a subject, and optimize a transmit parameter of an ultrasound or compensate for a receive parameter according to the subject (human body) features.

[Summary]

In accordance with some embodiments of the present disclosure, an ultrasonic medical apparatus includes an impulse generator, a transducer, an analyzer and an optimizer. The impulse generator is configured to generate an impulse. The transducer is configured to transmit the impulse to a subject and receive a first echo signal reflected in response to the impulse. The analyzer is configured to generate a subject attribute or a subject feature information by using frequency characteristics of the first echo signal and generate an analysis result data by comparing the subject feature information with a default value. The optimizer is configured to optimize, based on the analysis result data, at least one transmit parameter of an ultrasound to be transmitted to the subject, or compensate, based on the analysis result data, for at least one receive parameter of a second echo signal received in response to the ultrasound from the subject.

Further, in accordance with some embodiments of the present disclosure, an ultrasonic medical apparatus includes an impulse generator, a transducer, an analyzer, a signal processor, a transmitter and a scan converter. The impulse generator is configured to generate an impulse. The transducer is configured to transmit the impulse to a subject and receive a first echo signal reflected in response to the impulse. The analyzer is configured to identify resonance frequency characteristics of at least one transducer element based on the first echo signal. The signal processor is configured to generate an arbitrary waveform reflecting the resonance frequency characteristics and generate a normalized signal by normalizing the arbitrary waveform. The transmitter is configured to generate an ultrasound based on the normalized signal. The scan converter is configured to convert a second echo signal received in response to the ultrasound from the subject into an image data for display and to render the image data to be displayed on a display unit.

Further, in accordance with some embodiments of the present disclosure, an ultrasonic optimization method performed by an ultrasonic medical apparatus includes

generating an impulse,

transmitting the impulse to a subject and receiving a first echo signal reflected in response to the impulse,

performing an analysis, including generating a subject attribute or feature information by using frequency characteristics of the first echo signal, and generating an analysis result data by comparing the subject feature information with a default value, and

performing an optimization, including optimizing, based on the analysis result data, at least one transmit parameter of an ultrasound to be transmitted to the subject, or compensating, based on the analysis result data, for at least one receive parameter of a second echo signal received in response to the ultrasound from the subject.

Further, in accordance with some embodiments of the present disclosure, an ultrasonic optimization method performed by an ultrasonic medical apparatus includes

generating an impulse,

transmitting the impulse to a subject and receiving a first echo signal reflected in response to the impulse,

performing an analysis, including identifying resonance frequency characteristics of at least one transducer element based on the first echo signal;

performing a signal processing, including generating an arbitrary waveform reflecting the resonance frequency characteristics, and generating a normalized signal by normalizing the arbitrary waveform,

generating an ultrasound based on the normalized signal, and

transmitting the ultrasound to the subject.

[Advantageous Effects]

At least one embodiment of the present disclosure as described above, can identify subject (human body) features by analyzing an echo signal corresponding to an impulse transmitted to the subject, and it can optimize a transmit parameter of an ultrasound or compensate for a receive parameter according to the subject (human body) features. In other words, without an operator's direct adjustment of an image parameter or manual input of a parameter with a view to optimizing an ultrasound image, the ultrasound can be optimized in accordance with human body features by analyzing an echo signal corresponding to the impulse.

Moreover, at least one embodiment of the present disclosure as described above can optimize an ultrasound image by adjusting a parameter of a transmitted ultrasound or a received reflection signal depending on the subject (human body) features instead of adjusting a parameter value for an image. More specifically, some embodiments of the present disclosure optimize the formed image by adjusting the transmit parameter of the ultrasound transmitted to the subject or compensates for the receive parameter of the reflected signal corresponding to the ultrasound. In addition, at least one embodiment of the present disclosure as described above can prevent a deviation in the ultrasound image due to different subject (human body) features.

[Brief Description of Drawings]

FIG. 1 is a schematic block diagram of an ultrasonic medical apparatus according to a first apparatus embodiment of the present disclosure.

FIG. 2 is a schematic block diagram of a signal processor of an ultrasonic medical apparatus according to a second apparatus embodiment of the present disclosure.

FIG. 3 is a flowchart of an ultrasonic optimization method utilizing subject (human body) features according to a first method embodiment of the present disclosure.

FIG. 4 is a flowchart of an ultrasonic optimization method utilizing resonance frequency characteristics according to a second method embodiment of the present disclosure.

FIGs. 5A and 5B are diagrams of a method for obtaining subject (human body) features or resonance frequency characteristics by using an impulse according to the first and second method embodiments of the present disclosure.

FIG. 6 is a diagram of a transmit pulser for obtaining realtime images by using a resonance frequency according to the second method embodiment of the present disclosure.

FIG. 7 is a diagram of a signal processing process for generating an arbitrary waveform for a frequency displacement according to the second method embodiment of the present disclosure.

FIG. 8 is a diagram of a process for optimizing a transmit parameter or compensating for a receive parameter by utilizing the subject (human body) features according to the first method embodiment of the present disclosure.

FIG. 9 is a diagram of a system according to the first method embodiment of the subject (human body) features.

REFERENCE NUMERALS

| | |
|---|---|
| 100: ultrasonic medical apparatus | 110: transducer |
| 120: transmission/reception switch | 130: impulse generator |
| 132: transmitter | 134: receiver |
| 140: beamformer | 150: analog-digital converter |
| 162: analyzer | 164: optimizer |
| 170: signal processor | 180: scan converter |
| 190: display unit | |

[Detailed Description]

Hereinafter, the first and second apparatus embodiments of the present disclosure will be described in detail with reference to the accompanying drawings.

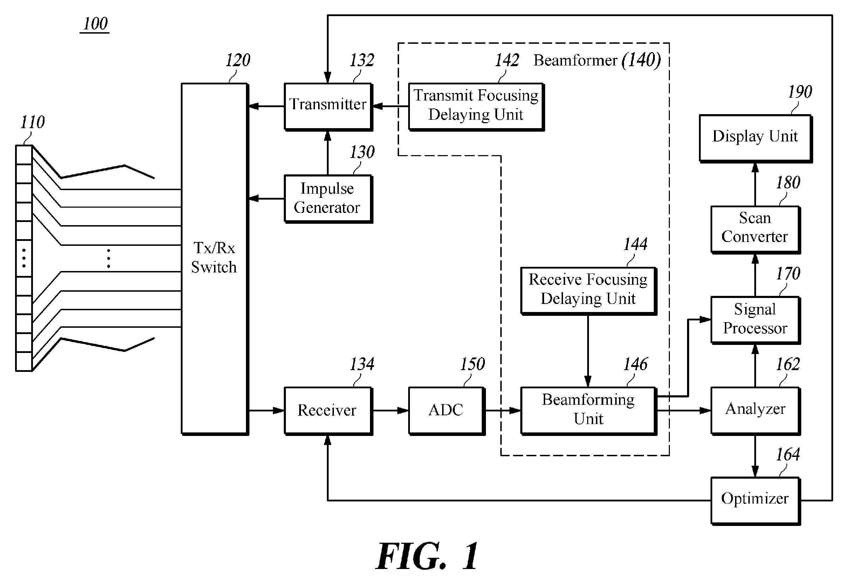

FIG. 1 is a schematic block diagram of an ultrasonic medical apparatus according to a first embodiment of the present disclosure.

According to the first apparatus embodiment, an ultrasonic medical apparatus 100 includes a transducer 110, a transmission/reception switch 120, an impulse generator 130, a transmitter 132, a receiver 134, a beamformer 140, an analog-digital converter 150, an analyzer 162, an optimizer 164, a signal processor 170, a scan converter 180 and a display unit 190. In the present embodiment, the ultrasonic medical apparatus 100 is described to include only the transducer 110, transmission/reception switch 120, impulse generator 130, transmitter 132, receiver 134, beamformer 140, analog-digital converter 150, analyzer 162, optimizer 164, signal processor 170, scan converter 180 and display unit 190, but is not necessarily limited thereto. Alternatively, various modifications, additions, and substitutions to constituent elements included in the ultrasonic medical apparatus 100 are possible, without departing from the gist and the nature of the first embodiment of the present disclosure.

The transducer 110 converts an electric analog signal into an ultrasound, transmits the ultrasound to a subject, and receives a signal reflected at the subject (hereinafter, referred to as an echo signal), and converts the echo signal into an electric analog signal. In general, the transducer 110 includes a plurality of transducer elements coupled to each other. The transducer 110 converts an acoustic energy into an electric signal, and vice versa. In some embodiments, the transducer 110 includes a transducer array, transmits the ultrasound to the subject by using transducer elements in the transducer array, and receives an echo signal from the subject.

The transducer 110 may include a plurality of (e.g., 128) transducer elements, and generates an ultrasound in response to a voltage applied from the transmitter 132. In this case, part of the transducer elements may be used to transmit the ultrasound. For example, when the transducer 110 includes 128 transducer elements, only 64 transducer elements may involve in the transmission of an ultrasound to form one transmit scanline. The transducer 110 can be used for both transmission and reception. The transducer 110 can be implemented with a plurality of 1 D (dimension), 1.25D, 1.5D, 1.75D or 2D transducer arrays.

The transducer 110 according to the first embodiment transmits an impulse to a subject (or a ROI, region of interest of the subject) selected by an operator, and receives a first echo signal corresponding to an impulse from the subject. In this case, the transducer 110 transmits the impulse to the subject when the operator inputs a command to transmit an impulse. Further, the transducer 110 transmits an ultrasound to the subject (or the ROI of the subject) selected by the operator, and receives a second echo signal corresponding to the ultrasound from the subject.

Further, the transducer 110 transmits an ultrasound focused by appropriately delaying the input times of pulses to the respective transducer elements under the control of the beamformer 140, to the subject along the transmit scanline. The second echo signal reflected from the subject in response to the ultrasound is fed to the transducer 110 at a different reception time, and the transducer 110 outputs the second echo signal to the beamformer 140.

The transmission/reception switch 120 serves to switch connections to/from the transmitter 132 and the receiver 134 so that the transducer 110 may alternately perform transmission and reception. In addition, the transmission/reception switch 120 serves to prevent a voltage outputted from the transmitter 132 from affecting the receiver 134.

The impulse generator 130 generates an impulse and transmits it to the transmitter 132 or directly applies the impulse to the transducer 110 under control of the transmission/reception switch 120 so that the impulse is output from each of the transducer elements of the transducer 110. Here, the impulse refers to a voltage or a current or a shock wave having a large amplitude within an extremely short time. The impulse generator 130 is illustrated at a location for connection with the transmission/reception switch 120 or the transmitter 132, but is not necessarily limited thereto.

Rather, the impulse generator 130 may be provided to be connected to another module within the ultrasonic medical apparatus 100.

The transmitter 132 includes a pulser which generates a pulse and applies it to the transducer 100. For example, the transmitter 132 applies a voltage pulse to the transducer 110, so that each transducer element of the transducer 110 outputs an ultrasound. Further, the transmitter 132 according to the first embodiment applies the impulse generated by the impulse generator 130 to the transducer 110, and all or part of the transducer elements of the transducer 110 outputs the impulse. The receiver 134 receives the returning first echo signal after the impulse outputted from each transducer element of the transducer 110 is reflected from the subject, processes the received first echo signal through at least amplification, aliasing phenomenon and noise component elimination, and correction of attenuation occurring while the impulse passes through a body, and transmits the processed signal to the analog-digital converter 150. Further, the receiver 134 receives the returning second echo signal after the ultrasound outputted from each transducer element of the transducer 110 is reflected from the subject, processes the received second echo signal through at least amplification, aliasing phenomenon and noise component elimination, and correction of attenuation occurring while the ultrasound passes through a body, and transmits the processed signal to the analog-digital converter 150.

The beamformer 140 appropriately delays electric signals for the transducer 110, to convert the electric signals into an electric signal suitable for each transducer element. Further, the beamformer 140 delays or sums the electric signals converted by respective transducer elements, to calculate an output value of the corresponding transducer element. The beamformer 140 includes a transmit beamformer, a receive beamformer and a beamforming unit 146. Here, the transmit beamformer is represented in FIG. 1 by a transmit focusing delaying unit 142, and the receive beamformer is represented by a receive focusing delaying unit 144. Further, the beamformer 140 generates a delay time required to focus the ultrasound on the subject, and then generates a combined signal by combining digital signals to which the delay time is applied.

The transmit focusing delaying unit 142 adds an appropriate delay to each electric digital signal in consideration of the time to reach each transducer element from the subject to be diagnosed. That is, when the transducer 110 includes a transducer array, the transmit focusing delaying unit 142 adjusts the beam and electronically focuses the beam. The transducer array is supposed to electronically focus the beam according to different depths, while the transmit focusing delay unit 142 focuses the beam on the transmission side by successively applying a pulse delay time to each of the transducer elements of the transducer array. Consequently, the transmit focusing delaying unit 142 adjusts the direction of the beam with respect to the transducer array that is electronically scanned. The receive focusing delaying unit 144 generates a delay time required to focus the digital signal converted by the analog-digital converter 150 or to perform a beamforming. That is, the receive focusing delaying unit 144 provides a time delay for focusing the echo signal received from the transducer 110, and adjusts a dynamic focusing of the echo signal.

The beamforming unit 146 forms a receive focusing signal by summing the electrical digital signals converted by the analog-to-digital converter 150. The beamforming unit 146 combines the digitized signals into a single signal. At this time, echo signals having the same phase are coupled by the beamforming unit 146, and after being subject to various signal processing methods by the signal processing unit 170, outputted by the display unit 190 via the scan converter 180. The beamforming unit 146 applies different amounts of delay (that depend on where the receive focusing is desired) to the signals received from the analog-to-digital converter 150, and performs the dynamic focusing by combining delayed signals. That is, the beamforming unit 146 combines the echo signals respectively received from the transducer elements into a single signal for a subsequent signal processing. The beamforming unit 146 generates a combined signal upon combining the echo signals received from the transducer elements, in order to form a single echo signal for each reflecting member (subject). The combined signal generated in this manner is transmitted from the beamforming unit 146 to the signal processor 170, and finally transmitted to a digitizing device that converts the combined signal into a digital signal for image data storage.

The analog-digital converter 150 converts an analog echo signal into a digital signal and then transmits the digital signal to the beamforming unit 146. The echo signal as received by the analog-to-digital converter 150 from the transducer 110 takes the form of analogue signal that is represented by a continuous voltage signal. In this case, the analog signal needs to be converted to a digital signal prior to being processed by the scan converter 180. Therefore, the analog-to-digital converter 150 converts the analog form of echo signal into a combination of 0s and 1 s. The digitized binary signal from the analog-to-digital converter 150 goes through the signal processor 170 and is stored in the memory of the scan converter 180. Further, the analog-digital converter 150 converts the first echo signal or the second echo signal into the digital signal.

The analyzer 162 generates a subject attribute or a subject feature information by using frequency characteristics of the first echo signal received in response to the impulse, and then generates an analysis result data by comparing the subject feature information with a default value. In this case, the default value is a kind of reference value and may be set to a cumulative average of different corresponding feature information, an experimentally determined optimum value or an ideal value. Here, the subject feature information includes a plurality of pieces of coefficient information, which will be described in detail below. That is, the analyzer 162 identifies an information on one or more from among a reflection coefficient, a transmission coefficient, an acoustic impedance, a scattering coefficient, an attenuation coefficient, a modulus of elasticity, and a temperature coefficient by using the frequency characteristics of the first echo signal, and recognizes the information as the subject feature information.

The analyzer 162 transmits to the optimizer 164 the information (analysis result data) needed for the optimizer 164 to optimize the transmit parameter or compensate for the receive parameter. The analyzer 162 calculates an amount of attenuation in the subject based on the attenuation coefficient ($\alpha$), an ultrasound propagation distance (d) and an ultrasound frequency, calculates a speed of sound (c) in a medium based on the density (p) and the stiffness (B) of the medium, and calculates the reflection coefficient (R) based on a ratio of a reflected sound pressure amplitude ($P_r$) and an incident sound pressure amplitude ($P_i$). The attenuation coefficient $\alpha$, ultrasound propagation distance d, frequency, attenuation amount, medium density p, stiffness B, speed of sound c, sound pressure amplitude $P_r$, sound pressure amplitude $P_i$ and reflection coefficient (R) may be transmitted to the optimizer 164 by the analyzer 162 as the information (analysis result data) for determining whether to optimize the transmit parameter or to compensate for the receive parameter.

In line with this, an operation of the analyzer 162 will now be described below.

By measuring ultrasound propagation distance d, the analyzer 162 may calculate attenuation coefficients $\alpha$ for respective bodily tissues using [Equation 1], and calculate the attenuation amount. The analyzer 162 may calculate the amount of the attenuation of the impulse passing through the thickness of a tissue of the subject (human body) by multiplying the attenuation coefficient (dB/cm) by the ultrasound propagation distance (cm).

$$\text{Attenuation amount [dB]} = \text{attenuation coefficient [dB/(cm MHz)]} \\ \times \text{ultrasound propagation distance [cm]} \\ \times \text{frequency [MHz]}$$

Equation 1

The impulse is attenuated within the tissue of the subject. The amplitude or intensity of the impulse passing through the tissue of the subject is decreased depending on its propagation distance. The attenuation is typically represented by even more decreasing amplitudes in proportion to increasing propagation distances.

The attenuation is caused by refraction, scattering, or absorption on a boundary surface of the tissue of the subject. The refraction and the scattering of the impulse within the tissue of the subject cause the attenuation, and refraction at a specific location (e.g., tumor tissue or the like) may be responsible for an ultrasound loss. However, when the subject is a large organ, the refraction and the scattering do not seemingly exercise a profound impact, but contribute to the overall attenuation. Further, acoustic energy is converted into thermal energy by absorption that causes the attenuation within the tissue of the subject (human body). The acoustic energy means a loss.

The attenuation coefficient within the subject (human body) is typically represented by 'dB/cm'. In this case, the impulse needs to reciprocate between reflectors, and therefore it moves a distance corresponding to two times the interval between the reflectors. When the reflectors are equal (i.e., have the same magnitude, the same direction, and the same reflection coefficient), their echo signals have the same signal magnitude. The attenuation coefficient of the tissue represented by 'dB/cm' is obtained by quantifying the change in signal magnitude.

The tissue of the subject (human body) may be one of soft tissues, for which tests have been conducted with respective attenuation coefficients, to exhibit the following reflection coefficients of tissues: water '0.0002 dB/cm', blood '0.18 dB/cm', liveer '0.5 dB/cm', and muscle '1.2 dB/cm'. The reflection coefficients were measured at the frequency of 1 MHz. At this frequency, the attenuation coefficient of the water is very low, the attenuation coefficient of a multiorgan parenchyma (such as liver) is moderate, and the attenuation coefficient of the muscle is slightly high. In the meantime, the attenuation coefficient in the soft tissue has a value between 0.5 dB/cm and 1 dB/cm at the frequency of 1 MHz.

The attenuation in the soft tissue is closely related to the frequency of the ultrasound. In most cases, the attenuation is substantially proportional to the frequency. Accordingly, when the attenuation coefficient of the tissue at the frequency of 1 MHz is given and the frequency becomes 2 MHz, the attenuation coefficient is also doubled. When the frequency is 5 MHz, the attenuation coefficient quintuples. As the frequency is increased, the attenuation is increased, so that the depth of penetration of the ultrasound into the tissue becomes shallow. That is, when an image is acquired by using the highest ultrasound frequency, the best spatial resolution is obtained.

Moreover, the analyzer 162 may determine a rate of change in volume (V) when a pressure is applied to the subject (human body) by using Equation 2 and Equation 3 below, thereby determining the stiffness B.

$$c = \sqrt{\frac{B}{\rho}} \ [m/s]$$

Equation 2

(c: speed of sound, B: stiffness, p: density of medium)

$$B = -V\frac{\partial P}{\partial V} = \rho\frac{\partial P}{\partial \rho} \qquad [N/m^2]$$

Equation 3

(B: stiffness, p: density of medium, P: power, V: volume, $\alpha$: attenuation coefficient)

That is, the analyzer 162 may determine varying densities p according to the subject (human body) when analyzing the first echo signal of the impulse corresponding to that would be transmitted by the transducer 110, by the same distance of the transducer 110 from the same subject and at the same position and pressure condition thereof.

Further, the analyzer 162 may determine the reflection coefficient R by using Equation 4 below, and determines an acoustic impedance Z of the subject (e.g., human body) by using the reflection coefficient R.

$$R = \frac{P_r}{P_i} = \frac{Z_2 - Z_1}{Z_2 + Z_1}$$

Equation 4

(R: reflection coefficient, $P_i$: incident sound pressure amplitude, $P_r$: reflected sound pressure amplitude, Z: impedance wherein $Z_1$ is the acoustic impedance of an adjacent first medium (proximal medium) and $Z_2$ is the acoustic impedance of a second medium (interfacial medium))

That is, after identifying the acoustic impedance, the analyzer 162 may predict scattering of an ultrasound transmit (Tx) signal, and thereafter predicts a noise component. The information may be used when the optimizer 164 compensates for the receive parameter. After the analyzer 162 analyzes the first echo signal corresponding to the impulse transmitted to the subject, a frequency characteristic and a pulse shape are changed by the attenuation and the impedance depending on the frequency, and an ultrasound transmission velocity varies for respective frequencies. In this case, when the component has a higher frequency, the transmission velocity is higher, so that the analyzer 162 may determine the rate of change according to the subject (human body) at the ultrasound velocity of the boundary surface according to the tissue of the subject.

Further, the analyzer 162 identifies a subject feature information based on a center frequency and a frequency displacement of the first echo signal received in response to the impulse. The analyzer 162 obtains an acoustic impedance information based on a time axis to predict a non-linear component of the subject. The analyzer 162 generates an analysis result data which includes a super default value information indicating that a coefficient is larger than the default value or a sub-default value information indicating that the coefficient is equal to or smaller than the default value by comparing a plurality of coefficients included in the first echo signal respectively with the default value.

The optimizer 164 optimizes the transmit (Tx) parameter of the ultrasound to be transmitted to the subject or compensates for the receive (Rx) parameter of the second echo signal received in response to the ultrasound from the subject based on the analysis result data received from the analyzer 162. That is, the optimizer 164 calculates an added score by assigning a weight to each of the coefficients (the reflection coefficient, the transmission coefficient, the acoustic impedance, the scattering coefficient, the attenuation coefficient, the modulus of elasticity, the temperature coefficient, and the like) included in the analysis result data received from the analyzer 162, and adjusts the transmit parameter or the receive parameter according to the added score. For example, the optimizer 164 applies a predetermined weight to each of the coefficients (the reflection coefficient, the transmission coefficient, the acoustic impedance, the scattering coefficient, the attenuation coefficient, the modulus of elasticity, the temperature coefficient, and the like) included in the analysis result data, calculates an added score by adding all the coefficients each of which the weight is applied to, and then adjusts the transmit parameter or the receive parameter under a condition corresponding to the added score. In addition, the optimizer 164 adjusts the transmit parameter or the receive parameter corresponding to the super default value information of the coefficient information included in the analysis result data to be reduced by the weight or adjusts the transmit parameter or the receive parameter corresponding to the sub-default value information of the coefficient information to be increased by the weight.

The transmit parameter optimized by the optimizer 164 is as follows. The optimizer 164 adjusts one or more of the transmit parameters from among an apodization parameter differently setting a size of each transducer element depending on a focal point, a transmit burst parameter determining the number of times of transmitting the ultrasound, a transmit frequency selection parameter selecting a center frequency of a transmit (Tx) waveform, and an aperture compounding parameter allowing realtime images to be displayed by differently setting a ratio of the received signal according to each time difference frame based on the analysis result data.

Further, the receive parameter compensated by the optimizer 164 is as follows. The optimizer 164 adjusts one or more of the receive parameters from among a signal size changing parameter changing a size of the received signal based on a time axis, a filter selection parameter selecting a filter (e.g., a low pass filter (LPF), a high pass filter (HPF)) required for image processing, an image setting parameter setting an image data according to the subject, and a receive frequency selection parameter selecting a center frequency of a receive (Rx) waveform based on the analysis result data.

The signal processor 170 converts the echo signals of receive scanlines, focused by the beamforming unit 146 to baseband signals, detects an envelope by using a quadrature demodulator to obtain data for a single scanline. Furthermore, signal processor 170 performs the A/D conversion of the data generated by the beamformer 140 into a digital signal.

The scan converter 180 records the data obtained by the signal processor 170 into memory, directs the data scanning to match the pixel direction of the display unit 190 (i.e., monitor), and maps the corresponding data to the pixel positions on the display unit 190. The scan converter 180 converts the ultrasound image data into a data format for use in the display unit 190 having a predetermined scanline display format.

The primary role of the scan converter 180 is to store temporary ultrasound image data. The scan converter 180 receives an echo signal from the transducer 110, and then stores the echo signal received in the internal memory (i.e., storage device). Then, the scan converter 180 converts the echo signal into image data and outputs the same on the display unit 190. In this case, image data may be converted into B-mode image data as well as M-mode image data, Doppler mode image data and color flow mode image data. If the scan converter 180 is not in the stop mode, the echo signal stored in the internal memory is constantly updated to be new information. Here, the converted image data is output to the display unit 190 as the same data is updated in real time. On the other hand, in the stop mode, the scanning operation is stopped, and the scan converter 180 performs only the output function. The scan conversion of the scan converter 180 is required because the format of the image acquisition is different from that of its reconstruction, and the ultrasound image data is output on the display unit 190. In this case, the echo signals reach the scan converter 180 along the respective scanlines. In addition, the memory of the scan converter 180 takes a buffer role between different data formats while writing and reading the data. The scan converter 180 receives the echo signal at the information format and the speed of the transducer 110. The scan converter 180 records the echo signal as a unit of image data in the memory. The image data is read from the memory by the scan converter 180 for display on the display unit 190 or monitor and is processed to conform to the horizontal image scanning by the display unit 190.

The memory of the scan converter 180 can be recognized as a matrix of elements, each made up of multi-bit storage units with respect to the ultrasound image data received from a preset position. Here, the digitized element is referred to as a pixel. That is, the memory of the scan converter 180 is a matrix of such pixels. Ultrasound image data that is output on the display unit 190 is actually present in the memory of the scan converter 180, in a matrix form of digital numbers. During a probing operation, the echo signal is inserted in the pixel position (address) depending on the location of the object. In order to calculate the exact pixel addresses, the scan converter 180 uses the delay time of the echo signal and beam coordinates of the transducer 110.

At this time, to present the value of the echo signal on the position of each pixel, the scan converter 180 operates on at least eight bits. An 8-bit has 256 amplitude levels at each position. Such memory of the scan converter 180 is constantly updated with new echo signal information as the ultrasonic beam proceeds to the ROI. Meanwhile, the image stop function of the scan converter 180 enables the echo signal to be stored in the memory for not only image recording but also photo or other digital information storage. The memory of the scan converter 180 provides its output by transmitting the values of the pixels to the digital-analog converter (DAC) for supplying the necessary signals to adjust the level of brightness of the display unit 190.

The display unit 190 outputs the ultrasound image data generated by the scan converter 180. Ultrasound image data described in some embodiments includes a B-mode image and a C-mode image. The B-mode image is a grayscale image in an image mode for displaying a motion of a target object, and the C-mode image is an image in a color flow image mode. A BC-mode image is an image in an image mode for displaying a blood flow or a motion of the target object by using a Doppler effect, which simultaneously provides the B-mode image and the C-mode image to provide anatomic information together with the blood flow and the motion of the target object. That is, the B-mode is a grayscale image mode for displaying the motion of the target object, and the C-mode is a color flow image mode for displaying the blood flow or the motion of the target object. An ultrasound medical apparatus 100 according to some embodiments is capable of simultaneously providing a B-mode image and the C-mode image that is a color flow image.

In some embodiments, the ultrasound medical apparatus 100 further includes a user input unit which receives an instruction from an operation or an input of a user. In some embodiments, the user instruction includes a setting instruction for controlling the ultrasound medical apparatus 100 and the like.

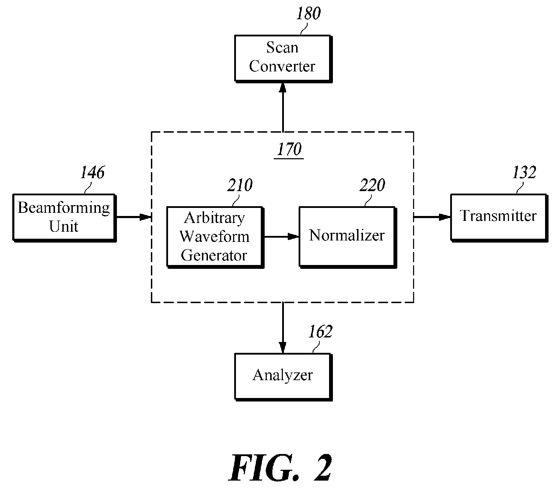

FIG. 2 is a schematic block diagram of a signal processor of an ultrasonic medical apparatus according to a second embodiment of the present disclosure.

The signal processor 170 according to the second embodiment includes an arbitrary waveform generator 210 and a normalizer 220. In the second embodiment, the signal processor 170 is described to include only the arbitrary waveform generator 210 and the normalizer 220, but is not necessarily limited. Therefore, various modifications, additions, and substitutions to constituent elements included in the signal processor 170 are possible, without departing from the gist and the nature of the second of the present disclosure.

The analyzer 162 identifies resonance frequency characteristics of the transducer element based on the first echo signal received in response to the impulse. Here, the resonance frequency refers to a resonance frequency of the transducer element (piezoelectric element) of the transducer 110. At the resonance frequency, electric energy is most efficiently converted into acoustic energy, and vice versa. In general, the resonance frequency may be mainly set by a thickness of the piezoelectric element of the transducer 110. For example, a piezoelectric element having a small thickness has a high resonance frequency, and a thick piezoelectric element may be set to have a low resonance frequency. The transducer 110 operates at the resonance frequency of the piezoelectric element or a frequency close thereto, so that a thickness of a piezoelectric element attached to a transducer for a high frequency is smaller than that of a piezoelectric element attached to a transducer for a low frequency.

For example, a broadband transducer is designed to operate at one or more frequencies, and a frequency of an acoustic wave emitted when the transducer is used may be determined by the ultrasonic medical apparatus 100 itself. The operator may select a frequency used for flaw detection by using a selection switch of the ultrasonic medical apparatus 100, and a transmitter of the ultrasonic medical apparatus 100 determines an electric pulse applied to the transducer 110 in order to generate the selected frequency. In this case, an amplifier of the receiver is also adjusted to have the same frequency.

However, since the resonance frequency set by the thickness of the piezoelectric element of the transducer 110 includes thermal noise according to the frequency characteristics of the transmit pulse, the analyzer 162 according to the second embodiment exactly identifies the resonance frequency of the transducer element based on the first echo signal received in response to the impulse.

The arbitrary waveform generator 210 generates an arbitrary waveform with the resonance frequency characteristics reflected therein. The normalizer 220 generates a normalized signal by processing the arbitrary waveform through a fast Fourier transform (FFT) and then a normalization. Here, the FFT is spectral analysis in which a complex signal is divided into simple frequency components or is analyzed. The most common method used for the spectral analysis is Fourier analysis.

To perform the spectral analysis in the ultrasonic medical apparatus 100, the normalizer 220 included in the signal processor 170 performs the FFT. The normalizer 220 represents a plurality of frequency components expressed by a function of time by a Doppler signal. The normalizer 220 consecutively divides a segment of the Doppler signal into small pieces of about 1 to 5 ms. In this case, the segment of the signal is converted into a digital value by the analog-digital converter 150 and sent to the normalizer 220. The normalizer 220 presents respective relative signal values at several discrete frequencies, then processes other signal segments, and continually presents the processed signal segments. As a result of performing the FFT by the normalizer 220, a horizontal axis represents a time and is divided into small intervals corresponding to the segments of the signal. In other words, a vertical axis represents a Doppler frequency or a velocity of a reflector and is divided into discrete frequencies to be stored. The larger the value stored at vertical intervals is, the higher the frequency is. The normalizer 220 has a gray concentration or density for representing a frequency distribution between the segments. The segments of the signal are successfully divided, thereby enabling the analyzer to represent a continuous spectrum. The normalization performed by the normalizer 220 refers to a process of digitizing an unnormalized signal (signal subjected to the FFT). That is, the normalization refers to a process of setting a reference with respect to the unnormalized signal, identifying a peak, and calculating a $'\pi'$.

The transmitter 132 according to the second embodiment generates an ultrasound based on a normalized signal received from the normalizer 220. That is, the transmitter 132 according to the second embodiment includes a pulser that generates an ultrasound based on the normalized signal. Thereafter, the transducer 11 according to the second embodiment transmits the ultrasound generated based on the normalized signal to the subject, and receives a second echo signal corresponding to the ultrasound generated based on the normalized signal from the subject. Then, the scan converter 180 receives the second echo signal corresponding to the ultrasound generated based on the normalized signal, and converts the second echo signal into an (untrasonic) image data for displaying the second echo signal, so that the image data is displayed on the display unit included therein.

In other words, the impulse generator 130 of the ultrasonic medical apparatus 100 according to the second embodiment generates an impulse, the ultrasonic medical apparatus 100 transmits the impulse from the transducer 110 to the subject, and then receives a first echo signal received in response to the impulse. Then, the analyzer 162 of the ultrasonic medical apparatus 100 identifies resonance frequency characteristics of the transducer element based on the first echo signal, the arbitrary waveform generator 210 generates an arbitrary waveform with the resonance frequency characteristics reflected therein, and the normalizer 220 generates a normalized signal by normalizing the arbitrary waveform and transmits the normalized signal to the transmitter 132. Then, the transmitter 132 of the ultrasonic medical apparatus 100 generates an ultrasound based on the normalized signal received from the normalizer 220. The transducer 110 of the ultrasonic medical apparatus 100 transmits the ultrasound generated based on the normalized signal to the subject, and receives a second echo signal corresponding to the ultrasound generated based on the normalized signal from the subject. Then, the scan converter 180 of the ultrasonic medical apparatus 100 receives the second echo signal, and converts the second echo signal into an (ultrasonic) image data for displaying the second echo signal, so that the image data is displayed on the display unit 190 included therein. That is, the resonance frequency characteristics of the transducer element of the transducer 110 have been already reflected to the ultrasound generated based on the normalized signal, and as a result, the optimized (ultrasonic) image data is generated in the scan converter 180.

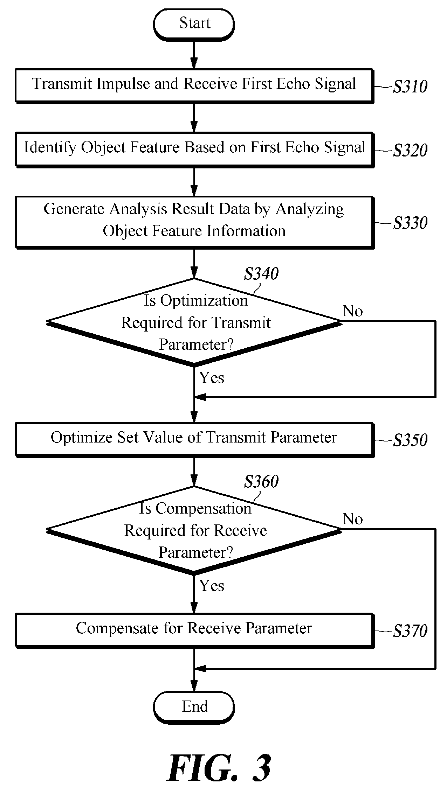

FIG. 3 is a flowchart of an ultrasonic optimization method utilizing subject (human body) features according to the first embodiment of the present disclosure.

The ultrasonic medical apparatus 100 generates an impulse, transmits the impulse to the subject, and receives a first echo signal reflected in response to the impulse from the subject (S310). The ultrasonic medical apparatus 100 identifies a subject feature information by using frequency characteristics of the first echo signal received in response to the impulse (S320). In step S320, the ultrasonic medical apparatus 100 identifies an information on one or more from among a reflection coefficient, a transmission coefficient, an acoustic impedance, a scattering coefficient, an attenuation coefficient, a modulus of elasticity, and a temperature coefficient by using the frequency characteristics of the first echo signal and recognizes the information as the subject feature information. In this case, the ultrasonic medical apparatus 100 identifies the subject feature information based on a center frequency and a frequency displacement of the first echo signal received in response to the impulse.

The ultrasonic medical apparatus 100 generates an analysis result data by comparing the subject feature information with a default value (S330). In step S330, the ultrasonic medical apparatus 100 generates the analysis result data which includes a super default value information indicating that a coefficient is larger than the default value or a sub-default value information indicating that the coefficient is equal to or smaller than the default value by comparing a plurality of coefficients included in the first echo signal respectively with the default value. Further, in step S330, the ultrasonic medical apparatus 100 calculates an amount of attenuation in the subject based on the attenuation coefficient ($\alpha$), an ultrasound propagation distance (d) and a frequency, calculates a speed of sound (c) based on a density (p) of a medium and a stiffness (B), and calculates the reflection coefficient (R) based on a ratio of a reflected sound pressure amplitude ($P_r$) and an incident sound pressure amplitude ($P_i$). The attenuation coefficient ($\alpha$), the ultrasound propagation distance (d), the frequency, the amount of the attenuation, the density (p) of the medium, the stiffness (B), the speed of sound (c), the sound pressure amplitude ($P_r$), the sound pressure amplitude ($P_i$), and the reflection coefficients (R) may be generated as the information (analysis result data) for determining whether to optimize the transmit parameter or compensate for the receive parameter by the ultrasonic medical apparatus 100. In addition, the ultrasonic medical apparatus 100 may predict a non-linear component of the subject by obtaining an acoustic impedance information based on a time axis.

The ultrasonic medical apparatus 100 checks whether optimization is required for the transmit parameter of the ultrasound to be transmitted to the subject based on the analysis result data (S340). In step S340, the ultrasonic medical apparatus 100 calculates an added score by assigning a weight to each of the coefficients (the reflection coefficient, the transmission coefficient, the acoustic impedance, the scattering coefficient, the attenuation coefficient, the modulus of elasticity, the temperature coefficient, and the like) included in the analysis result data, and checks whether optimization is required for the transmit parameter according to the added score. For example, the ultrasonic medical apparatus 100 applies a predetermined weight to each of the coefficients (the reflection coefficient, the transmission coefficient, the acoustic impedance, the scattering coefficient, the attenuation coefficient, the modulus of elasticity, the temperature coefficient, and the like) included in the analysis result data, calculates the added score by adding all the coefficients each of which the weight is applied to, and when there is an optimization condition corresponding to the added score, adjusts (optimizes) the transmit parameter.

As a result of checking in step S340, when optimization is required for the transmit parameter, the ultrasonic medical apparatus 100 optimizes the transmit parameter according to the corresponding condition (S350). That is, in step S350, the ultrasonic medical apparatus 100 adjusts the transmit parameter corresponding to the super default value information of the coefficient information included in the analysis result data to be reduced by the weight, or adjusts the transmit parameter corresponding to the sub-default value information of the coefficient information to be increased by the weight. In this case, the ultrasonic medical apparatus 100 adjusts one or more of the transmit parameters from among an apodization parameter differently setting a size of each transducer element depending on a focal point, a transmit burst parameter determining the number of times of transmitting the ultrasound, a transmit frequency selection parameter selecting a center frequency of a transmit waveform, and an aperture compounding parameter allowing realtime images to be displayed by differently setting a ratio of the received signal according to each time difference frame based on the analysis result data.

The ultrasonic medical apparatus 100 checks whether compensation is required for the receive parameter of the second echo signal received in response to the ultrasound from the subject (S360). In step S360, the ultrasonic medical apparatus 100 calculates an added score by assigning a weight to each of the coefficients (the reflection coefficient, the transmission coefficient, the acoustic impedance, the scattering coefficient, the attenuation coefficient, the modulus of elasticity, the temperature coefficient, and the like) included in the analysis result data, and checks whether compensation is required for the receive parameter according to the added score. For example, the ultrasonic medical apparatus 100 applies a predetermined weight to each of the coefficients (the reflection coefficient, the transmission coefficient, the acoustic impedance, the scattering coefficient, the attenuation coefficient, the modulus of elasticity, the temperature coefficient, and the like) included in the analysis result data, calculates the added score by adding all the coefficients each of which the weight is applied to, and when there is a compensation condition corresponding to the added score, adjusts (compensates for) the receive parameter.

As a result of checking in step S360, when compensation is required for the receive parameter, the ultrasonic medical apparatus 100 compensates for the receive parameter according to the corresponding condition (S370). That is, in step S370, the ultrasonic medical apparatus 100 adjusts the receive parameter corresponding to the super default value information of the coefficient information included in the analysis result data to be reduced by the weight, or adjusts the receive parameter corresponding to the sub-default value information of the coefficient information to be increased by the weight. In this case, the ultrasonic medical apparatus 100 adjusts one or more of the receive parameters from among a signal size changing parameter changing a size of the received signal based on a time axis, a filter selection parameter selecting a filter (e.g., LPF, HPF) required for image processing, an image setting parameter setting an image data according to the subject, and a receive frequency selection parameter selecting a center frequency of a receive waveform based on the analysis result data.

Although steps S310 to S370 are described to be sequentially performed in the example shown in FIG. 3, they merely instantiate a technical idea of the first embodiment of the present disclosure. Therefore, a person having ordinary skill in the pertinent art could appreciate that various modifications, additions, and substitutions are possible by changing the sequences described in FIG. 3 or by executing one or more steps from S310 to S370 in parallel, without departing from the gist and the nature of the first embodiment of the present disclosure, and hence FIG. 3 is not limited to the illustrated chronological sequences.

The ultrasonic optimization method utilizing subject (human body) features according to the first embodiment shown in FIG. 3 can be implemented as a computer program, and can be recorded on a computer-readable recording medium. The computer-readable recording medium on which the program for implementing the ultrasonic optimization method utilizing subject (human body) features according to the first embodiment is recordable includes any type of recording device on which data that can be read by a computer system are recordable. Examples of the computer-readable recording medium are a ROM, a RAM, a CD-ROM, a magnetic tape, a floppy disk, an optical data storage device, and the like, and also include one implemented in the form of a carrier wave (e.g., transmission through the Internet). Further, the computer-readable recording medium can be distributed in computer systems connected via a network, and computer-readable codes can be stored and executed in a distributed mode. Moreover, functional programs, codes, and code segments for implementing the first embodiment can be easily deduced by a programmer in technical fields to which the first embodiment belongs.

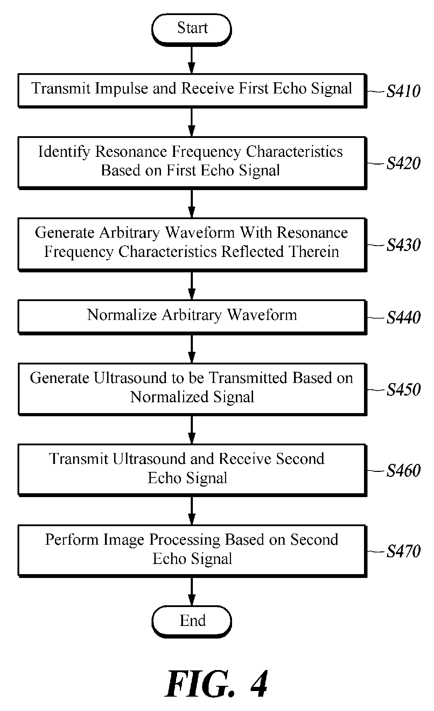

FIG. 4 is a flowchart of an ultrasonic optimization method utilizing resonance frequency characteristics according to the second embodiment of the present disclosure.

The ultrasonic medical apparatus 100 generates an impulse, transmits the impulse to the subject, and receives a first echo signal reflected in response to the impulse from the subject (S410). The ultrasonic medical apparatus 100 identifies resonance frequency characteristics by using frequency characteristics of the first echo signal received in response to the impulse (S420).

The ultrasonic medical apparatus 100 generates an arbitrary waveform with the resonance frequency characteristics reflected therein (S430). The ultrasonic medical apparatus 100 generates a normalized signal by processing the arbitrary waveform through a fast Fourier transform (FFT) and then a normalization (S440). The ultrasonic medical apparatus 100 generates an ultrasound based on the normalized signal (S450).

The ultrasonic medical apparatus 100 transmits the ultrasound generated based on the normalized signal to the subject, and receives a second echo signal corresponding to the ultrasound generated based on the normalized signal from the subject (S460). The ultrasonic medical apparatus 100 receives the second echo signal, and converts the second echo signal into an image data for displaying the second echo signal, so that the image data is displayed on the display unit included therein (S470). In other words, in the ultrasonic medical apparatus 100, the resonance frequency characteristics of the transducer element of the transducer 110 have been already reflected to the ultrasound generated based on the normalized signal, and as a result, the optimized (ultrasonic) image data is generated in the scan converter 180.

Although steps S410 to S470 are described to be sequentially performed in the example shown in FIG. 4, they merely instantiate a technical idea of the second embodiment of the present disclosure. Therefore, a person having ordinary skill in the pertinent art could appreciate that various modifications, additions, and substitutions are possible by changing the sequences described in FIG. 4 or by executing one or more steps from S410 to S470 in parallel, without departing from the gist and the nature of the second embodiment of the present disclosure, and hence FIG. 4 is not limited to the illustrated chronological sequences.

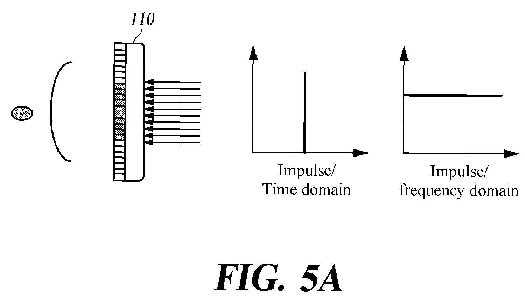

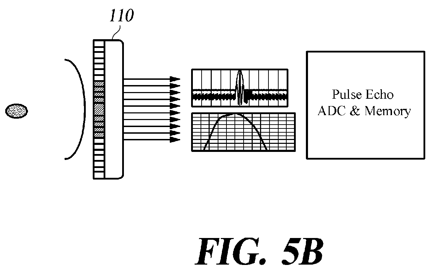

FIGs. 5A and 5B are diagrams of a method for obtaining subject (human body) features or resonance frequency characteristics by using an impulse, according to the first and second embodiments of the present disclosure.

The impulse generator 130 of the ultrasonic medical apparatus 100 generates the impulse. In this case, the impulse generator 130 transmits the impulse to the transmitter 132, or directly applies the impulse to the transducer 110 under control of the transmission/reception switch 120, so that each transducer element of the transducer 110 outputs the impulse. That is, the transducer 110 of the ultrasonic medical apparatus 100 transmits the impulse to the subject, and receives the first echo signal corresponding to the impulse from the subject. Then, the analyzer 162 of the ultrasonic medical apparatus 100 identifies the resonance frequency characteristics of the transducer element of the transducer 110 and applies the resonance frequency characteristics to the generation of the ultrasound as it is.

As illustrated in FIG. 5A, when an operator selects a 'test mode' or a 'subject (human body) feature identification mode', the ultrasonic medical apparatus 100 transmits an impulse to the subject in order to identify resonance frequency characteristics of the transducer element of the transducer 110. As illustrated in FIG. 5B, the ultrasonic medical apparatus 100 receives a first echo signal corresponding to the impulse from the subject, subjects the first echo signal to an analog-digital conversion, and stores the converted signal in a memory.

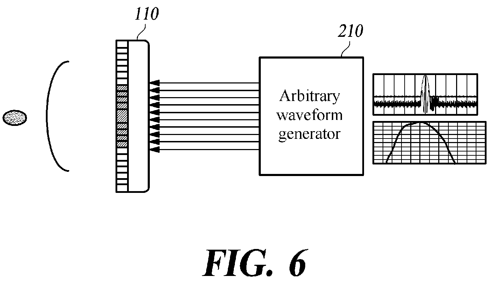

FIG. 6 is a diagram of a transmit pulser for obtaining realtime images by using a resonance frequency according to the second embodiment of the present disclosure.

When the operator selects a 'realtime mode', the ultrasonic medical apparatus 100 generates an ultrasound for obtaining realtime images by using an optimal resonance frequency obtained by analyzing the first echo signal received in response to the impulse. As illustrated in FIG. 6, the ultrasonic medical apparatus 100 identifies resonance frequency characteristics of the transducer element based on the first echo signal received in response to the impulse. The ultrasonic medical apparatus 100 generates an arbitrary waveform with the resonance frequency characteristics reflected therein by using the arbitrary waveform generator 210. That is, the ultrasonic medical apparatus 100 identifies the optimal resonance frequency characteristics of the transducer 110, and generates the ultrasound transmitted to the subject with the frequency, thereby generating an ultrasound image data.

In this case, the ultrasonic medical apparatus 100 identifies the resonance frequency characteristics exactly coinciding with characteristics of the transducer element of the transducer 110, and in turn uses the resonance frequency characteristics to generate the ultrasound image data. Moreover, when the resonance frequency exactly coinciding with the characteristics of the transducer element of the transducer 110 is obtained and used to generate the ultrasound image data, a thermal noise generated in the transducer 110 may be minimized, and higher transmit (Tx) power may be applied to the subject (human body), thereby improving penetration.

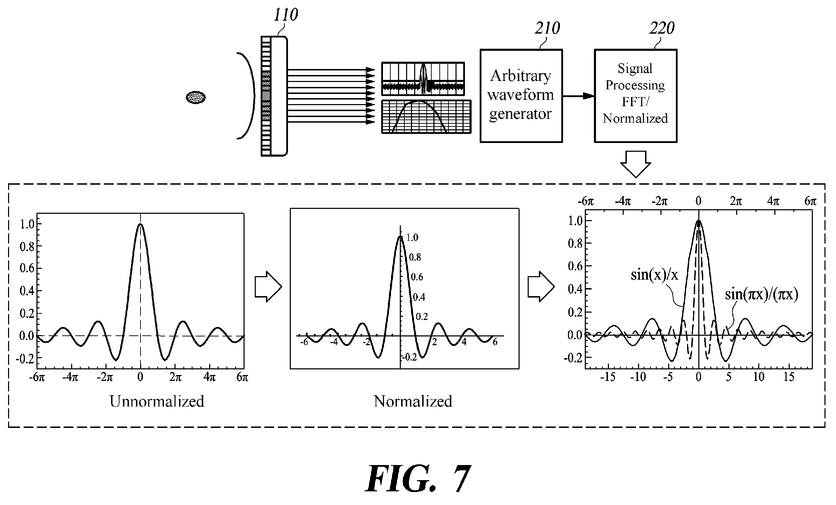

FIG. 7 is a diagram of a signal processing process for generating an arbitrary waveform for a frequency displacement according to the second embodiment of the present disclosure.

As illustrated in FIG. 7, the ultrasonic medical apparatus 100 generates a normalized signal by processing the arbitrary waveform through a fast Fourier transform (FFT) and then a normalization by using the normalizer 220. That is, the normalizer 220 of the ultrasonic medical apparatus 100 generates the arbitrary waveform with the resonance frequency characteristics reflected therein, and then uses the FFT for the best frequency displacement. The ultrasonic medical apparatus 100 identifies the optimal resonance frequency characteristics based on the first echo signal, and changes a frequency by the FFT and the normalization. The ultrasonic medical apparatus 100 may generate an ideal ultrasound for forming a harmonic image by performing the foregoing signal processing operation.

When the impulse generator 130 generates the impulse, the ultrasonic medical apparatus 100 transmits the impulse from the transducer 110 to the subject, and receives the first echo signal reflected in response to the impulse. Then, the analyzer 162 identifies resonance frequency characteristics of the transducer element based on the first echo signal, the arbitrary waveform generator 210 generates an arbitrary waveform with the resonance frequency characteristics reflected therein, and the normalizer 220 generates a normalized signal by normalizing the arbitrary waveform, and transmits the normalized signal to the transmitter 132. Then, the transmitter 132 generates an ultrasound based on the normalized signal received from the normalizer 220, the transducer 110 transmits the ultrasound based on the normalize signal to the subject, and receives a second echo signal corresponding to the ultrasound generated based on the normalized signal from the subject. Then, the ultrasonic medical apparatus 100 converts the second echo signal into an image data for displaying the second echo signal, so that the image data is displayed on the display unit included therein. In other words, the resonance frequency characteristics of the transducer element of the transducer 110 have been already reflected to the ultrasound generated based on the normalized signal, and as a result, the converted image data is optimized in the scan converter 180.

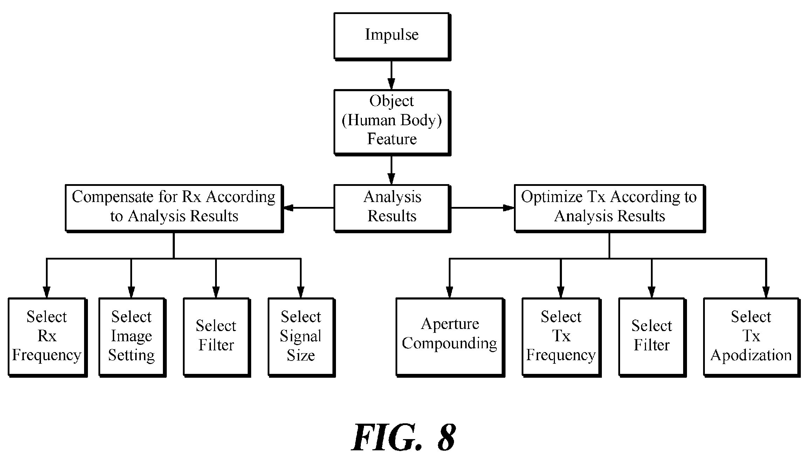

FIG. 8 is a diagram of a process for optimizing a transmit parameter or compensating for a receive parameter by utilizing the subject (human body) features according to the first embodiment of the present disclosure.

As illustrated in FIG. 8, the ultrasonic medical apparatus 100 identifies and analyzes subject (human body) features having different coefficients. In this case, the ultrasonic medical apparatus 100 transmits an impulse to the subject in order to identify the subject (human body) features, analyzes a first echo signal corresponding to the impulse (e.g., identifies a signal to noise ratio (SNR), a center frequency, or a point having a high frequency displacement), and optimizes a transmit parameter. Here, the transmit parameter optimizable by the ultrasonic medical apparatus 100 may be one or more of the parameters from among an apodization parameter differently setting a size of each transducer element depending on a focal point, a transmit burst parameter determining the number of times of transmitting the ultrasound, a transmit frequency selection parameter selecting a center frequency of a transmit waveform, and an aperture compounding parameter allowing realtime images to be displayed by differently setting a ratio of the received signal according to each time difference frame, as illustrated in FIG. 8.

Further, the ultrasonic medical apparatus 100 transmits the impulse to the subject in order to identify of the subject (human body) features, analyzes the first echo signal corresponding to the impulse (e.g., identifies a signal to noise ratio (SNR), a center frequency or a point having a high frequency displacement), and compensates for the receive parameter. Here, the receive parameter capable of being compensated by the ultrasonic medical apparatus 100 may be one or more of the parameters from among a signal size changing parameter changing a size of the received signal based on a time axis, a filter selection parameter selecting a filter (e.g., LPF, HPF) required for image processing, an image setting parameter setting an image data according to the subject, and a receive frequency selection parameter selecting a center frequency of a receive waveform based on the analysis result data, as illustrated in FIG. 8.

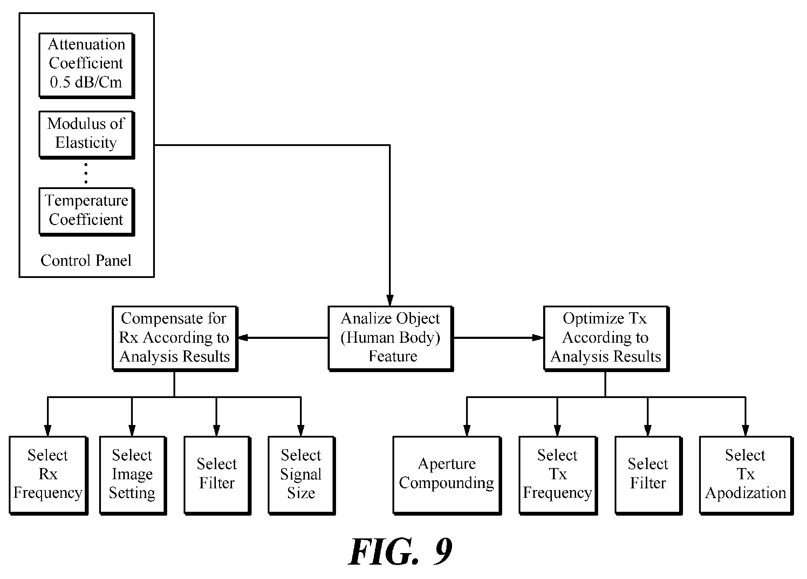

FIG. 9 is a diagram of a system according to the first embodiment of the subject (human body) features.

As illustrated in FIG. 9, the ultrasonic medical apparatus 100 selects or inputs a 'body information' of a patient from or to a control panel by a manipulation of the operator. That is, the ultrasonic medical apparatus 100 may generate a 'subject feature information' based on the 'body information' of the patient.

In the meantime, the ultrasonic medical apparatus 100 builds a database of accumulated subject feature information of a specific subject (patient), and sets the databased information as a reference value. For example, the ultrasonic medical apparatus 100 receives an information on at least one of a 'gender information', an 'age information', a 'height information', and a 'weight information' corresponding to the body information of the patient by the manipulation of the operator, extracts a transmit parameter or a receive parameter when an optimal ultrasound image data based on the body information is output, databases the transmit parameter or the receive parameter, and sets an average value obtained by accumulating a plurality of pieces of similar body information as a reference value.

Although exemplary embodiments of the present disclosure have been described for illustrative purposes, those skilled in the art will appreciate that various modifications, additions and substitutions are possible, without departing from the idea and scope of the claimed disclosure. Accordingly, one of ordinary skill would understand the scope of the claimed disclosure is not to be limited by the explicitly described above embodiments but by the claims and equivalents thereof.

CROSS-REFERENCE TO RELATED APPLICATION

If applicable, this application claims priority under 35 U.S.C §119(a) of Patent Application No. 10-2013-0052724, filed on May 9, 2013 in Korea, the entire content of which is incorporated herein by reference. In addition, this non-provisional application claims priority in countries, other than the U.S., with the same reason based on the Korean patent application, the entire content of which is hereby incorporated by reference.

**Claims**

1. An ultrasonic medical apparatus, comprising:

   an impulse generator configured to generate an impulse;
   a transducer configured to transmit the impulse to a subject and receive a first echo signal reflected in response to the impulse;
   an analyzer configured to generate a subject attribute or a subject feature information by using frequency characteristics of the first echo signal and generate an analysis result data by comparing the subject feature information with a default value; and

an optimizer configured to optimize, based on the analysis result data, at least one transmit parameter of an ultrasound to be transmitted to the subject, or compensate, based on the analysis result data, for at least one receive parameter of a second echo signal received in response to the ultrasound from the subject.

2. The ultrasonic medical apparatus of claim 1, wherein the analyzer is configured to:

   identify at least one information of a reflection coefficient, a transmission coefficient, an acoustic impedance, a scattering coefficient, an attenuation coefficient, a modulus of elasticity, and a temperature coefficient by using the frequency characteristics of the first echo signal; and
   recognize the at least one information as the subject feature information.

3. The ultrasonic medical apparatus of claim 2, wherein the analyzer is configured to:

   calculate an amount of attenuation in the subject based on the attenuation coefficient ($\alpha$), an ultrasound propagation distance (d) and a frequency of the ultrasound;
   calculate a speed of sound (c) in a medium based on a density (p) and a stiffness (B) of the medium; and
   calculate the reflection coefficient (R) based on a ratio of a reflected sound pressure amplitude ($P_r$) to an incident sound pressure amplitude ($P_i$).

4. The ultrasonic medical apparatus of claim 2, wherein the analyzer is configured to obtain the acoustic impedance information based on a time axis to predict a non-linear component of the subject.

5. The ultrasonic medical apparatus of claim 1, wherein the analyzer is configured to identify the subject feature information based on a center frequency and a frequency displacement of the first echo signal.

6. The ultrasonic medical apparatus of claim 1, wherein the analyzer is configured to:

   generate a super default value information in the analysis result data if the default value is exceeded in a comparison by each of one or more coefficient information included in the first echo signal; and
   generate a sub-default value information in the analysis result data if each of the one or more coefficient information is smaller than the default value.

7. The ultrasonic medical apparatus of claim 1, wherein the optimizer is configured to adjust, based on the analysis result data, one or more of the transmit parameters comprising an apodization parameter for differently setting the size of each element of the transducer depending on a focal point, a transmit burst parameter for determining the number of times of transmitting the ultrasound, a transmit frequency selection parameter for selecting a center frequency of a transmit waveform, and an aperture compounding parameter for allowing realtime images to be displayed by differently setting a ratio of a received signal according to each time difference frame.

8. The ultrasonic medical apparatus of claim 1, wherein the optimizer is configured to adjust, based on the analysis result data, one or more of the receive parameters comprising a signal size changing parameter for changing the size of a received signal based on a time axis, a filter selection parameter for selecting a filter required for an image processing, an image setting parameter for setting an image data according to the subject, and a receive frequency selection parameter for selecting a center frequency of a receive waveform.

9. The ultrasonic medical apparatus of claim 1, wherein the optimizer is configured to:

   calculate a weighted sum by assigning a weight to each of coefficients included in the analysis result data; and
   adjust the transmit parameter or the receive parameter according to the weighted sum.

10. The ultrasonic medical apparatus of claim 1, wherein the optimizer is configured to:

    adjust a transmit parameter or a receive parameter corresponding to super-default information out of the coefficient information included in the analysis result data to be reduced by a weight, or
    adjust a transmit parameter or a receive parameter corresponding to sub-default information out of the coefficient information to be increased by a weight.

11. The ultrasonic medical apparatus of claim 1, further comprising:

a signal processor configured to generate an arbitrary waveform reflecting the resonance frequency characteristics; and

a transmitter configured to generate the ultrasound based on the arbitrary waveform, and

wherein the analyzer is configured to identify resonance frequency characteristics of at least one of the elements of the transducer based on the first echo signal.

12. The ultrasonic medical apparatus of claim 11, wherein the signal processor comprises:

an arbitrary waveform generator configured to generate the arbitrary waveform reflecting the resonance frequency characteristics; and

a normalizer configured to perform a fast Fourier transform (FFT) and a normalization successively on the arbitrary waveform to generate a normalized signal, and

wherein the transmitter is configured to generate the ultrasound based on the normalized signal.

13. The ultrasonic medical apparatus of claim 12, further comprising a scan converter configured to:

convert the second echo signal into an image data for display; and

render the image data to be displayed on a display unit.

14. An ultrasonic diagnostic apparatus, comprising:

an impulse generator configured to generate an impulse;

a transducer configured to transmit the impulse to a subject and receive a first echo signal reflected in response to the impulse;

an analyzer configured to identify resonance frequency characteristics of at least one transducer element based on the first echo signal;

a signal processor configured to generate an arbitrary waveform reflecting the resonance frequency characteristics and generate a normalized signal by normalizing the arbitrary waveform;

a transmitter configured to generate an ultrasound based on the normalized signal; and

a scan converter configured to convert a second echo signal received in response to the ultrasound from the subject into an image data for display and to render the image data to be displayed on a display unit.

15. An ultrasonic optimization method performed by an ultrasonic medical apparatus, the method comprising:

generating an impulse;

transmitting the impulse to a subject and receiving a first echo signal reflected in response to the impulse;

performing an analysis, comprising:

generating a subject attribute or feature information by using frequency characteristics of the first echo signal, and

generating an analysis result data by comparing the subject feature information with a default value; and

performing an optimization, comprising:

optimizing, based on the analysis result data, at least one transmit parameter of an ultrasound to be transmitted to the subject; or

compensating, based on the analysis result data, for at least one receive parameter of a second echo signal received in response to the ultrasound from the subject.

16. The ultrasonic optimization method of claim 15, wherein the performing of the analysis comprises:

identifying at least one information of a reflection coefficient, a transmission coefficient, an acoustic impedance, a scattering coefficient, an attenuation coefficient, a modulus of elasticity and a temperature coefficient by using the frequency characteristics of the first echo signal; and

recognizing the at least one information as the subject feature information.

**17.** The ultrasonic optimization method of claim 15, wherein the performing of the optimization comprises:

calculating a weighted sum by assigning a weight to each of the coefficients included in the analysis result data; and

adjusting the transmit parameter or the receive parameter according to the weighted sum.

**18.** An ultrasonic optimization method performed by an ultrasonic medical apparatus, the method comprising:

generating an impulse;

transmitting the impulse to a subject and receiving a first echo signal reflected in response to the impulse;

performing an analysis, comprising:

identifying resonance frequency characteristics of at least one transducer element based on the first echo signal;

performing a signal processing, comprising:

generating an arbitrary waveform reflecting the resonance frequency characteristics, and generating a normalized signal by normalizing the arbitrary waveform;

generating an ultrasound based on the normalized signal; and

transmitting the ultrasound to the subject.

**FIG. 1**

EP 2 995 259 A1

*180*

Scan
Converter

*146*

Beamforming
Unit

*170*

*210*

Arbitrary
Waveform
Generator

*220*

Normalizer

*132*

Transmitter

*162*

Analyzer

# FIG. 2

```
                        ┌──────────┐
                        │  Start   │
                        └────┬─────┘
                             │
                             ▼
        ┌───────────────────────────────────────────┐
        │ Transmit Impulse and Receive First Echo Signal │──S310
        └────────────────────┬──────────────────────┘
                             │
                             ▼
        ┌───────────────────────────────────────────┐
        │ Identify Object Feature Based on First Echo Signal │──S320
        └────────────────────┬──────────────────────┘
                             │
                             ▼
        ┌───────────────────────────────────────────┐
        │  Generate Analysis Result Data by Analyzing  │──S330
        │       Object Feature Information            │
        └────────────────────┬──────────────────────┘
                             │        S340
                             ▼
                    ◇──────────────◇
                   ╱ Is Optimization ╲        No
                  ◇ Required for Transmit ◇──────────┐
                   ╲   Parameter?    ╱               │
                    ◇──────────────◇                │
                             │ Yes                    │
                             ▼◄───────────────────────┘
        ┌───────────────────────────────────────────┐
        │      Optimize Set Value of Transmit Parameter │──S350
        └────────────────────┬──────────────────────┘
                             │        S360
                             ▼
                    ◇──────────────◇
                   ╱ Is Compensation ╲       No
                  ◇ Required for Receive ◇──────────┐
                   ╲   Parameter?    ╱               │
                    ◇──────────────◇                │
                             │ Yes                    │
                             ▼                        │
        ┌───────────────────────────────────────────┐│
        │        Compensate for Receive Parameter     │──S370
        └────────────────────┬──────────────────────┘│
                             │◄───────────────────────┘
                             ▼
                        ┌──────────┐
                        │   End    │
                        └──────────┘
```

# FIG. 3

Start

Transmit Impulse and Receive First Echo Signal — S410

Identify Resonance Frequency Characteristics
Based on First Echo Signal — S420

Generate Arbitrary Waveform With Resonance
Frequency Characteristics Reflected Therein — S430

Normalize Arbitrary Waveform — S440

Generate Ultrasound to be Transmitted Based on
Normalized Signal — S450

Transmit Ultrasound and Receive Second
Echo Signal — S460

Perform Image Processing Based on Second
Echo Signal — S470

End

*FIG. 4*

*110*

Impulse/
Time domain

Impulse/
frequency domain

# FIG. 5A

110

Pulse Echo
ADC & Memory

# FIG. 5B

*110*

*210*

Arbitrary
waveform
generator

**FIG. 6**

**FIG. 7**

EP 2 995 259 A1

```
                          ┌─────────────┐
                          │   Impulse   │
                          └──────┬──────┘
                                 │
                                 ▼
                          ┌─────────────┐
                          │   Object    │
                          │(Human Body) │
                          │   Feature   │
                          └──────┬──────┘
                                 │
                                 ▼
┌──────────────────────┐  ┌─────────────┐  ┌──────────────────────┐
│Compensate for Rx     │◄─│  Analysis   │─►│Optimize Tx According  │
│According to Analysis  │  │   Results   │  │to Analysis Results    │
│Results                │  └─────────────┘  └──────────────────────┘
└──────────┬───────────┘                    └──────────┬───────────┘
```

| Select Rx Frequency | Select Image Setting | Select Filter | Select Signal Size | | Aperture Compounding | Select Tx Frequency | Select Filter | Select Tx Apodization |
|---|---|---|---|---|---|---|---|---|

## FIG. 8

Attenuation
Coefficient
0.5 dB/Cm

Modulus of
Elasticity

⋮

Temperature
Coefficient

Control Panel

Compensate for
Rx According to
Analysis Results

Analize Object
(Human Body)
Feature

Optimize Tx
According to
Analysis Results

Select
Rx
Frequency

Select
Image
Setting

Select
Filter

Select
Signal
Size

Aperture
Compounding

Select
Tx
Frequency

Select
Filter

Select
Tx
Apodization

*FIG. 9*

## INTERNATIONAL SEARCH REPORT

| International application No. |
| --- |
| **PCT/KR2013/004126** |

### A. CLASSIFICATION OF SUBJECT MATTER

*A61B 8/14(2006.01)i*

According to International Patent Classification (IPC) or to both national classification and IPC

### B. FIELDS SEARCHED

Minimum documentation searched (classification system followed by classification symbols)

A61B 8/14; G01S 5/18; G02N 29/22; A61N 1/18; A61B 18/00; A61N 7/00; A61B 8/00; A61N 7/02; G01N 29/24

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: transducer, impulse, reflective signal, frequency, default, analyzing unit, optimization, parameter

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2012-0123034 A (THERACLION SA) 07 November 2012<br>See abstract, paragraphs [0026]-[0038], [0049]-[0057] and figures 1-5. | 1,5,6,8,9,15,17 |
| Y | | 2-4,16 |
| A | | 7,10-14,18 |
| Y | KR 10-2007-0065332 A (GUIDED THERAPY SYSTEMS, L.L.C.) 22 June 2007<br>See pages 11-15 and figures 6A-6C. | 2-4,16 |
| A | | 1,5-15,17,18 |
| A | US 5684243 A (GURURAJA, Turuvekere R. et al.) 04 November 1997<br>See abstract, column 4, line 10 - column 6, line 25 and figures 3-13. | 1-18 |
| A | JP 2004-514461 A (ACUSON CORPORATION) 20 May 2004<br>See abstract, paragraphs [0008]-[0015] and figure 1. | 1-18 |
| A | KR 10-2008-0039634 A (SAMSUNG MEDISON CO.,LTD.) 07 May 2008<br>See abstract, paragraphs [0015]-[0025] and figure 1. | 1-18 |

☐ Further documents are listed in the continuation of Box C.   ☒ See patent family annex.

| | |
| --- | --- |
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>21 FEBRUARY 2014 (21.02.2014) | Date of mailing of the international search report<br><br>**24 FEBRUARY 2014 (24.02.2014)** |
| Name and mailing address of the ISA/KR<br>Korean Intellectual Property Office<br>Government Complex-Daejeon, 189 Seonsa-ro, Daejeon 302-701, Republic of Korea<br>Facsimile No. 82-42-472-7140 | Authorized officer<br><br><br>Telephone No. |

Form PCT/ISA/210 (second sheet) (July 2009)

INTERNATIONAL SEARCH REPORT
Information on patent family members

International application No.

**PCT/KR2013/004126**

| Patent document cited in search report | Publication date | Patent family member | Publication date |
|---|---|---|---|
| KR 10-2012-0123034 A | 07/11/2012 | EP 2332614 A1<br>EP 2512599 A1<br>KR 10-2012-0123034 A<br>US 2012-0238873 A1<br>WO 2011-069985 A1 | 15/06/2011<br>24/10/2012<br>07/11/2012<br>20/09/2012<br>16/06/2011 |
| KR 10-2007-0065332 A | 22/06/2007 | EP 1809377 A1<br>IL 181892 A<br>JP 2008-514294 A<br>JP 2012-050845 A<br>KR 10-1269918 B1<br>KR 10-2011-0091830 A<br>US 07530958 B2<br>US 2006-0074355 A1<br>US 2009-0216159 A1<br>WO 2006-036870 A1 | 25/07/2007<br>30/12/2010<br>08/05/2008<br>15/03/2012<br>31/05/2013<br>12/08/2011<br>12/05/2009<br>06/04/2006<br>27/08/2009<br>06/04/2006 |
| US 05684243 A | 04/11/1997 | JP 08-229036 A<br>US 05585546 A<br>US 05684243 A<br>US 05889194 A | 10/09/1996<br>17/12/1996<br>04/11/1997<br>30/03/1999 |
| JP 2004-514461 A | 20/05/2004 | JP 4847665 B2<br>WO 01-58357 A1 | 28/12/2011<br>16/08/2001 |
| KR 10-2008-0039634 A | 07/05/2008 | NONE | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130052724